# EUROPEAN PATENT APPLICATION

(11) **EP 3 978 579 A1**
(43) Date of publication of application: **06.04.2022**
(21) Application number: 19931411.3
(22) Date of filing: 17.12.2019
(51) Int. Cl.: C09J 175/04, C09J 201/00, B29C 65/48, G06F 30/23, G06F 30/10

(54) **ADHESIVE SELECTION METHOD, ADHESION COMPLEX, AND PRODUCTION METHOD FOR ADHESION COMPLEX**

(30) Priority: 28.05.2019 JP 2019099678
(71) Applicant: Sika Hamatite Co., Ltd., Kanagawa 254-0014 (JP)
(72) Inventor: ABE, Megumi, Hiratsuka-shi, Kanagawa 254-8601 (JP); MATSUKI, Yuichi, Hiratsuka-shi, Kanagawa 254-8601 (JP); HOU, Gang, Hiratsuka-shi, Kanagawa 254-8601 (JP)
(74) Representative: Dilg, Haeusler, Schindelmann Patentanwaltsgesellschaft mbH
(86) International application number: PCT/JP2019/049355
(87) International publication number: WO 2020/240900

(57) **Abstract**

Provided are an adhesive selection method which enables simple and suitable selection of an adhesive for bonding heterogeneous materials, an adhesion complex using the adhesive, and a production method for the adhesion complex.

Test data is acquired by subjecting an adhesion complex (1), obtained by integrally bonding one material (2) and the other material (3) that are heterogeneous using an adhesive (4), to a shear test, in which a tensile shear load is applied to the adhesion complex (1) in a direction that causes shear deformation in the adhesive (4), until adhesive failure takes place. Based on the test data, a plurality of two-dimensional or three-dimensional FEM analysis models (1M) of the adhesion complex (1) with varied adhesives (4a), (4b), (4c) are constructed. Then, on each of the plurality of analysis models (1Ma), (1Mb), (1Mc), a simulation is performed in which a tensile shear load is applied. Based on the stresses generated in each of the analysis models (1Ma), (1Mb), (1Mc) in the course of the simulation and the locations where the stresses are generated, the adhesive (4) for binding one member (2A) made of the one material (2) and the other member (3A) made of the other material (3) is selected from among the adhesives (4).

## Description

### Technical Field

The present invention relates to an adhesive selection method, an adhesion complex, and a production method for an adhesion complex, and particularly relates to: an adhesive selection method which enables simpler and more appropriate selection of an adhesive to be used while taking into consideration a process, experienced by an adhesion complex in which heterogeneous materials are bonded, which ends with adhesive failure; an adhesion complex using the adhesive; and a production method for an adhesion complex.

### Background Art

When selecting an adhesive to be used, bonding performance (such as adhesive strength and durability) of an adhesive with respect to the material to be bonded is taken into consideration. In the related art, various methods for analyzing bonding performance of adhesives have been proposed (for example, Patent Documents 1 and 2).

In recent years, there has been an increase of materials that can be used with an adhesive, and there have been more cases where heterogeneous materials are bonded together. In an adhesion complex in which heterogeneous materials are bonded together, different materials have different characteristics; thus, a process from an adhesion complex receiving external forces to adhesive failure of the adhesion complex is likely to be affected by the behaviors of the materials. The greater the difference in characteristics of the materials, the greater the effect on the process tends to be. Furthermore, in order to ensure the durability of the bond portion of an adhesion complex, taking into consideration the process which ends with adhesive failure is preferable. Therefore, there is room to improve methods in the related art regarding analyzing the bonding performance of an adhesive for bonding heterogeneous materials together and selecting a suitable adhesive.

### Citation List

### Patent Literature

Patent Document 1: JP 2016-148888 A
Patent Document 2: JP 2019-61601 A

### Summary of Invention

### Technical Problem

An object of the present invention is to provide: an adhesive selection method which enables simpler and more appropriate selection of an adhesive to be used while taking into consideration a process, experienced by an adhesion complex in which heterogeneous materials are bonded, which ends with adhesive failure; an adhesion complex using the adhesive; and a production method for an adhesion complex.

### Solution to Problem

In order to achieve the object described above, an adhesive selection method according to an embodiment of the present invention includes: acquiring test data by subjecting an adhesion complex, obtained by integrally bonding one material and an other material that are heterogeneous using an adhesive, to a shear test, in which a tensile shear load is applied to the adhesion complex in a direction that causes shear deformation in the adhesive, until adhesive failure takes place; constructing, based on the test data, a plurality of two-dimensional or three-dimensional FEM analysis models of the adhesion complex with varied adhesives; performing, on each of the plurality of analysis models, a simulation in which a tensile shear load is applied in a direction that causes shear deformation in the adhesive used in the analysis model; and selecting, from the adhesives, an adhesive for bonding the one material and the other material based on the tensile stresses generated in the analysis models in the course of the simulation and the locations where the tensile stresses are generated.

The adhesion complex according to an embodiment of the present invention includes an adhesive selected according to the adhesive selection method described above, one member made of the one material, and the other member made of the other material, the adhesive being interposed between the one member and the other member and integrating the one member and the other member.

The production method for an adhesion complex according to an embodiment of the present invention includes interposing an adhesive selected according to the adhesive selection method described above between one member made of the one material and the other member made of the other material to integrate the one member and the other member.

### Advantageous Effects of Invention

According to the adhesive selection method according to an embodiment of the present invention according to an embodiment of the present invention, test data is acquired by subjecting an adhesion complex, obtained by integrally bonding one material and an other material that are heterogeneous using an adhesive, to a shear test, in which a tensile shear load is applied to the adhesion complex in a direction that causes shear deformation in the adhesive, until adhesive failure takes place; based on the test data, a plurality of two-dimensional or three-dimensional FEM analysis models of the adhesion complex with varied adhesives are constructed. Then, on each of the plurality of analysis models, a simulation is performed in which a tensile shear load is applied in a direction that causes shear deformation in the adhesive used in the analysis model; data regarding the tensile stresses generated in the analysis models in the course of the simulation and the locations where the tensile stresses are generated is used. Thus, while the adhesive selection method according to an embodiment of the present invention adopts a simple simulation in which a tensile shear load is applied to each of the analysis models, it also enables selection of an adhesive while taking into consideration the process, experienced by an adhesion complex, which ends with adhesive failure. As a result, it is possible to suitably select an adhesive, among the adhesives used in the simulation, that can ensure bonding performance that meets requirements.

Furthermore, in the adhesion complex and the production method for an adhesion complex according to the present invention, a suitable adhesive selected in accordance with the selection method described above is used, making it possible to obtain an adhesion complex suitable for the use condition. Accordingly, it becomes advantageous to improve durability of the adhesion complex and trust in the durability.

### Brief Description of Drawings

- FIG. 1: is a perspective view illustrating an adhesion complex according to an embodiment of the present invention.
- FIG. 2: is an explanatory diagram illustrating a state where a test piece of the adhesion complex in FIG. 1 is placed in a shear tester in a side view.
- FIG. 3: is an explanatory diagram illustrating the test piece in FIG. 2 in a plan view.
- FIG. 4: is a graph diagram showing test data obtained from a shear test.
- FIG. 5: is an explanatory diagram illustrating an analysis model.
- FIG. 6: is an explanatory diagram illustrating a state where a tensile shear load is applied to the analysis model in FIG. 5.
- FIG. 7: is a graph diagram showing a relationship between the maximum tensile stress generated in an analysis model during a simulation and a tensile shear load.

### Description of Embodiments

An adhesive selection method, an adhesion complex, and a production method for an adhesion complex according to an embodiment of the present invention will be described below with reference to the drawings.

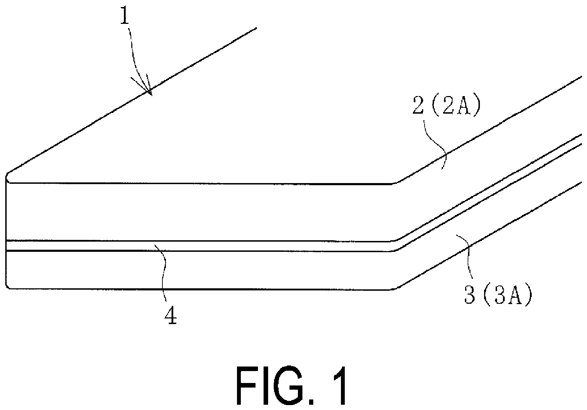

The adhesion complex 1 according to an embodiment of the present invention illustrated in FIG. 1 includes one member 2A made of one material 2, the other member 3A made of the other material 3, the one material 2 and the one material 3 being heterogeneous, and an adhesive 4 interposed between the one member 2A and the other member 3A, the members 2A and 3A being integrally bonded by the adhesive 4. The shape of the one member 2A and the other member 3A is not limited to a planar shape, and various shapes can be adopted. That is, the one member 2A is formed into a variety of shapes using the one material 2, and the other member 3A is formed into a variety of shapes using the other material 3.

Here, "heterogeneous materials" refers to not only materials different from each other (for example, resins of different types), but also materials that are identical to each other (for example, the identical type of resin) but differs in terms of, for example, the presence of reinforcing material mixed or embedded, or specifications of the reinforcing material. That is, materials having different mechanical properties (tensile elastic modulus, tensile strength, and the like) are deemed as heterogeneous materials.

The one material 2 to be used has, for example, a tensile elastic modulus of 500 MPa or greater and 2000 MPa or less and a tensile strength of 10 MPa or greater and 30 MPa or less. Examples of the one material 2 include various thermoplastic resins such as polypropylene and various thermosetting resins such as epoxy resins. In a configuration in which the one material 2 is polypropylene mixed with talc, the tensile elastic modulus is 2 GPa or less, the tensile strength is 18 MPa or less, and the Poisson's ratio is 0.4.

The other material 3 to be used has, for example, a tensile elastic modulus of 2000 MPa or greater and 10000 MPa or less and a tensile strength of 30 MPa or greater and 150 MPa or less. Examples of the other material 3 include materials in which reinforcing materials are mixed or embedded in various thermoplastic resins such as polypropylene or various thermosetting resins such as epoxy resins. The tensile elastic modulus of the other material 3 is, for example, two times or more, or three times or more, the tensile elastic modulus of the one material 2. In a configuration in which the other material 3 is polypropylene mixed with glass fibers, the tensile elastic modulus is 2.7 GPa or greater and 7.7 GPa or less, the tensile strength is 80 MPa or greater and 180 MPa or less, and the Poisson's ratio 0.4. The tensile elastic modulus, tensile strength, and Poisson's ratio of the one material 2 and the other material 3 are calculated in accordance with JIS K 7161.

The adhesive 4 to be used has, for example, a tensile elastic modulus of 1 MPa or greater and 25 MPa or less, a tensile strength (tensile adhesion strength) of 3 MPa or greater and 25 MPa or less, and a Poisson's ratio of from 0.4 to 0.5. These values are values when the adhesive 4 hardens and expresses sufficient adhesive strength. The tensile strength, tensile elastic modulus, and Poisson's ratio of the adhesive 4 are calculated in accordance with JIS K 6849.

The adhesive 4 can be any as long as it is one that adheres to the one material 2 and the other material 3. For example, in a configuration in which the main component of the one material 2 and the other material 3 is polyurethane, the adhesive 4 to be used can be a two-part polyurethane-based adhesive that includes a main agent containing a urethane prepolymer and a curing agent containing a polyol.

Specifically, when the main component of the one material 2 and the other material 3 is polyurethane, the two-part polyurethane-based adhesive 4 can be a composition in which the urethane prepolymer in the main agent contains at least a urethane prepolymer obtained by reacting a bifunctional or trifunctional polyether polyol having a weight average molecular weight of 1000 or greater and 10000 or less with 4,4,4-diphenylmethane diisocyanate at an isocyanate index (NCO/OH molar ratio) of 1.5 to 40 while the polyol in the curing agent contains at least either a trifunctional polyol having a weight average molecular weight of 1000 or less or a bifunctional polyol having a weight average molecular weight of 400 or less. Furthermore, when the adhesive 4 is a two-part polyurethane-based adhesive in which either the main agent or the curing agent contains at least carbon or calcium carbonate, the two-part polyurethane adhesive 4 can be a composition in which the main agent and the curing agent are mixed at an isocyanate index (NCO/OH molar ratio) of 1/0.1 to 1/0.9, the isocyanate index indicating the ratio of isocyanate groups in the main agent to hydroxy groups in the curing agent.

In this embodiment, a case in which the one material 2 is a resin (tensile elastic modulus of approximately 1.6 GPa) in which talc is mixed with polypropylene while the other material 3 is a resin (tensile elastic modulus of approximately 5.4 GPa) in which glass fibers are mixed with polypropylene is used as an example. Furthermore, an adhesive 4a (tensile elastic modulus of 2 MPa, tensile strength of 5.6 MPa, elongation at break of 497%, JIS A hardness of 50), an adhesive 4b (tensile elastic modulus of 40 MPa, tensile strength of 10.9 MPa, elongation at break of 180%, JIS A hardness of 80), and an adhesive 4c (tensile elastic modulus of 279 MPa, tensile strength of 15.0 MPa, elongation at break of 50% or less, JIS A hardness of 90 or greater) are used as examples of the adhesive 4.

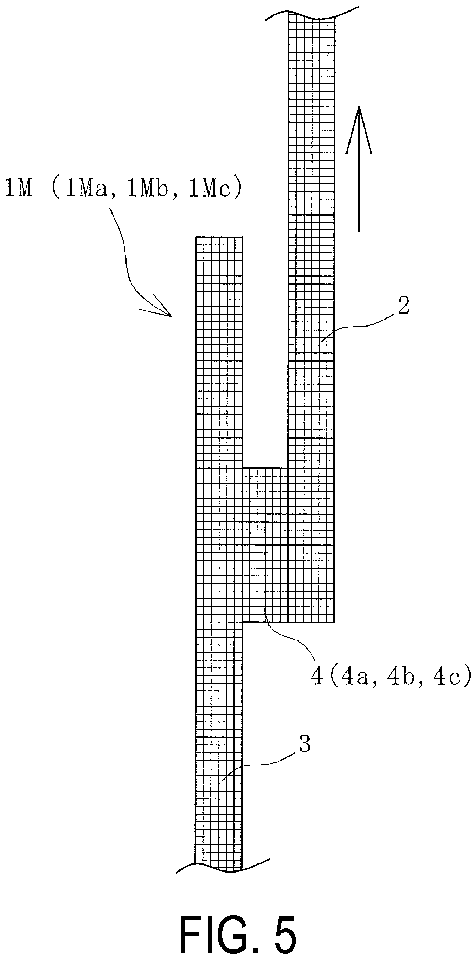

As illustrated in FIG. 5, the adhesive selection method according to an embodiment of the present invention uses a two-dimensional or three-dimensional FEM analysis model 1M of the adhesion complex 1 in which the one material 2 and the other material 3 are integrally bonded by the adhesive 4. The analysis model 1M is provided with constituents identical to the actual adhesion complex 1. In the drawings, each constituent of the analysis model 1M is labeled with reference signs identical to those of the constituents of the actual adhesion complex 1. A plurality of the analysis models 1M with varied adhesives 4 are constructed. Since the tensile elastic modulus of the adhesive 4 greatly affects the bonding performance of the adhesion complex 1, a plurality of the analysis models 1M (1Ma, 1Mb, 1Mc) using adhesives 4 (4a, 4b, 4c) with different tensile elastic moduli as described above are constructed. The adhesive 4 to be used in the analysis model 1M is not limited to three types, and a suitable number of types may be used.

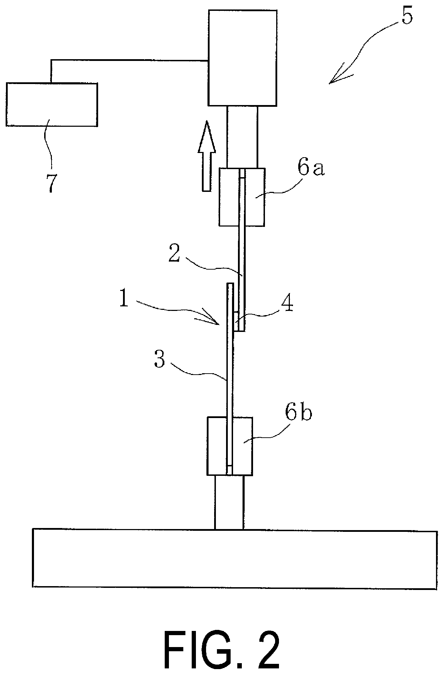

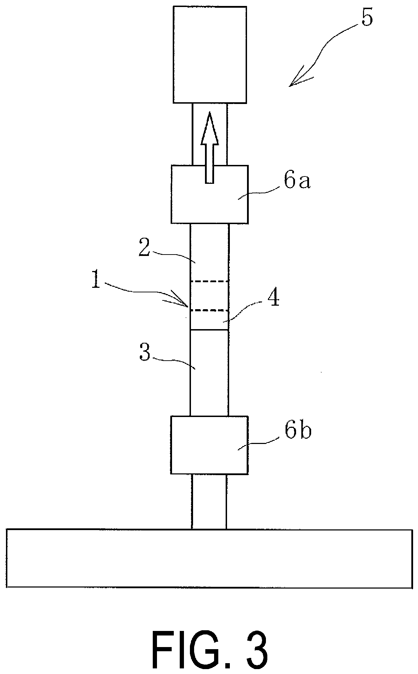

The analysis model 1M is constructed based on experimental data. Specifically, as illustrated in FIGS. 2 and 3, a shear test is performed using a shear tester 5 on a test piece of the adhesion complex 1 in which the one material 2 and the other material 3 are integrally bonded by the adhesive 4. In the adhesion complex 1, adhesive failure tends to occur when the adhesion complex 1 is subjected to external forces (shear forces) that are unaligned with the direction parallel to the opposing surfaces where the one member 2A and the other member 3A are bonded. Thus, a shear test in which a tensile shear load is applied in a direction that causes shear deformation in the adhesive 4 of the test piece of the adhesion complex 1 enables accurate evaluation of the bonding performance while simplifying the adhesive failure mode.

The shear tester 5 is provided with a pair of holders 6a, 6b and a computation device 7. The one holder 6a is movable in a direction towards and away from the other holder 6b, and the other holder 6b is fixed and unmovable relative to the base. The one material 2 of the test piece of the adhesion complex 1 is held by the one holder 6a, and the other material 3 of the test piece of the adhesion complex 1 is held by the other holder 6b. Then, the one holder 6a is moved in the direction away from the other holder 6b, the direction being indicated by the arrow, and a tensile shear load is applied in a direction that causes shear deformation in the adhesive 4. The shear test is performed until adhesive failure of the test piece takes place. That is, the shear test is performed until the adhesive portion and the periphery thereof (at least one of the one material 2, the other material 3, or the adhesive 4) fails, or until a substantial state of failure is reached.

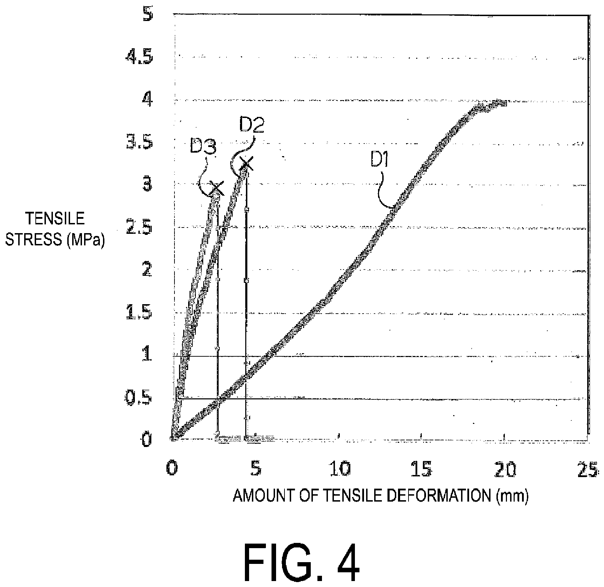

As shown in FIG. 4, during the shear test, test data (S-S curve data) of the tensile stress and the amount of tensile deformation generated in the test piece are measured and acquired by a known method and input and stored in the computation device 7 until adhesive failure of the test piece takes place. FIG. 4 shows test data D1, D2, D3 of three types of test pieces of the adhesive bonded article 1 in which only the adhesive 4 is varied while the one material 2 and the other material 3 remain unchanged as described above.

In each of the test pieces, the one material 2 and the other material 3 approximately have a length of 100 mm, a width of 25 mm, and a thickness of 3 mm, and the adhesive 4 approximately has an adhesion length of 10 mm, an adhesion width of 25 m, and a thickness of 3 mm. After a predetermined plasma treatment is performed on an adhering surface, the adhesive 4 is applied to the adhering surface. The tensile shear rate of the tensile shear load applied is set to 27 mm/min.

The test data D1, the data of the analysis model 1Ma using the adhesive 4a, has a maximum tensile stress of 4 MPa and an amount of tensile deformation of 20 mm at the maximum tensile stress point. At the time of maximum tensile stress, the one material 2 and the other material 3 have not failed, and severe tensile deformation are found in the adhesive 4a with cohesive failure observed. The test data D2, the data of the analysis model 1Mb using the adhesive 4b, has a maximum tensile stress of 3.2 MPa and an amount of tensile deformation of 4.46 mm at the maximum tensile stress point. At the time of maximum tensile stress, the one material 2 has not failed, and cohesive failure of the adhesive 4 has not occurred, but the other material 3 failed. The test data D3, the data of the analysis model 1Mc using the adhesive 4c, has a maximum tensile stress of 2.8 MPa and an amount of tensile deformation of 2.66 mm at the maximum tensile stress point. At the time of maximum tensile stress, the one material 2 has not failed, and cohesive failure of the adhesive 4 has not occurred, but the other material 3 failed.

In addition to the test data, physical property data (such as S-S curve data indicating tensile elastic modulus and tensile strength, and Poisson's ratio) and external data (such as length, thickness, width, and adhesion area) of the one material 2, the other material 3, and the adhesive 4 of the adhesion complex 1 to be subjected to a simulation are input to the computation device 7. Physical property data such as data of temperature dependency is also input if available.

Then, the analysis model 1M is constructed by a known method based on the data input to the computation device 7. It is easy to work with the analysis model 1M when it uses a solid model as in this embodiment. As illustrated in FIG. 5, a simulation is performed in which the lower end portion of the other material 3 of the analysis model 1M is fully constrained, and a tensile shear load is applied in a direction that causes shear deformation in the adhesive 4. The simulation can be performed while controlling the amount of the unidirectional upward movement of the upper end portion of the one material 2, or while controlling the amount of the tensile shear load applied to the upper end portion of the one material 2 in a unidirectional, upward manner.

The analysis result of the analysis model 1M constructed is compared with the test data (comparison of S-S curve data and the like) to confirm that there is little divergence between the two and that the analysis model 1M is applicable for practical use. Note that it is not necessary to acquire experimental data and construct an analysis model 1M for each specification of the adhesion complex 1. For example, as long as it is confirmed that an analysis model 1M constructed based on the test data of an adhesion complex 1 having a representative specification is applicable for practical use, the physical property data and the like of the adhesion complex 1 to be analyzed may be input to the analysis model 1M for use.

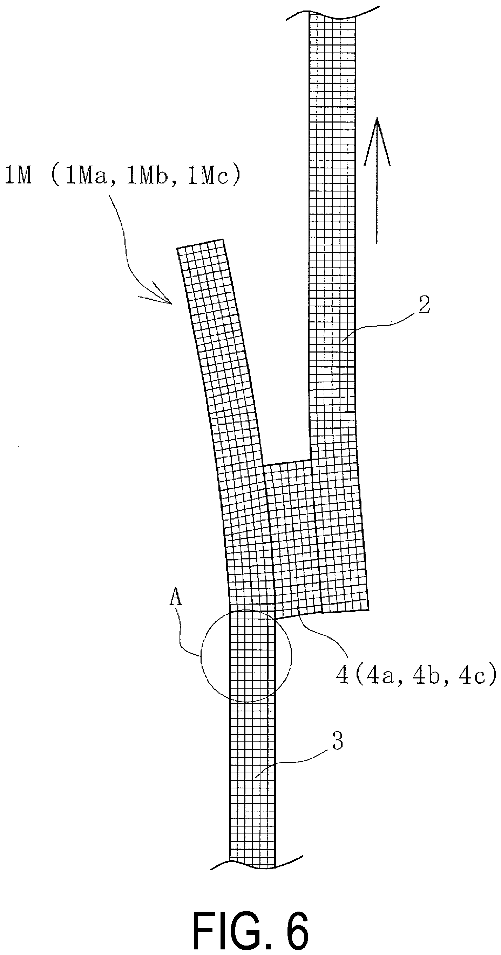

As illustrated in FIG. 6, during the simulation, the analysis model 1M is subjected to tensile deformation. In the course of the simulation, the tensile stress and the location of the tensile stress generated in each of the analysis models 1M are determined. Then, based on the results determined, a suitable adhesive for joining the one material 2 (the one member 2A) and the other material 3 (the other member 3A) is selected from the adhesives 4a, 4b, and 4c.

Since each analysis model 1M has a different specification, the tensile stress and the degree of deformation generated in the one material 2, the other material 3, and the adhesive 4 varies depending on the analysis model 1M. As such, for example, the adhesive 4 is selected based on a comparison between the maximum tensile stress, generated in the course of the simulation, in each of the one material 2, the other material 3, and the adhesive 4 of each of the analysis models 1M and the allowable tensile stress of each of the one material 2, the other material 3, and the adhesive 4.

In the analysis model 1M illustrated in FIG. 6, the tensile stress in each mesh is calculated, and thus, the location where the maximum tensile stress in each of the members is generated can be identified by confirming the mesh in which the maximum tensile stress in each of the members is generated. Then, in the course of the simulation, the tensile shear load applied to the analysis model 1M is calculated by the computation device 7 when the maximum tensile stress generated in the one material 2 exceeds the allowable tensile stress of the one material 2. Similarly, the tensile shear load applied to the analysis model 1M is calculated when the maximum tensile stress generated in the other material 3 exceeds the allowable tensile stress of the other material 3. Then, the tensile shear load applied to the analysis model 1M is calculated when the maximum tensile stress generated in the adhesive 4 exceeds the allowable tensile stress of the adhesive 4. The smallest value among the tensile shear loads calculated for each of the members is the tensile shear load that the analysis model 1M can withstand.

Next, the tensile shear loads that each of the analysis models 1M can withstand are compared, and it is determined that the higher the value of the tensile shear load that an analysis model 1M can withstand, the less likely adhesive failure will occur, and the better the bonding performance of the analysis model 1M. When it is determined that the analysis model having the best bonding performance is the analysis model 1Ma, followed by the analysis model 1Mb and the analysis model 1Mc in this order, the most suitable adhesive 4 to be selected is the adhesive 4a, followed by the adhesive 4b and the adhesive 4c in this order.

Alternatively, the adhesive 4 may be selected based on a comparison between the maximum tensile stress in each of the analysis models 1M generated in the course of the simulation and the allowable tensile stress of the member having the location where the maximum tensile stress is generated. In this case, in the course of the simulation, data of the location at which the maximum tensile stress is generated is acquired. In the analysis model 1M illustrated in FIG. 6, the tensile stress in each mesh is calculated, and thus, the location where the maximum tensile stress in the analysis model 1M is generated can be identified by confirming the mesh in which the maximum tensile stress is generated. That is, it is possible to determine at what location of the one material 2, the other material 3, and the adhesive 4 is the maximum tensile stress generated. In this embodiment, it is calculated that the maximum tensile stress is generated in part A, illustrated in FIG. 6, of the other material 3 in the entire course of the simulation.

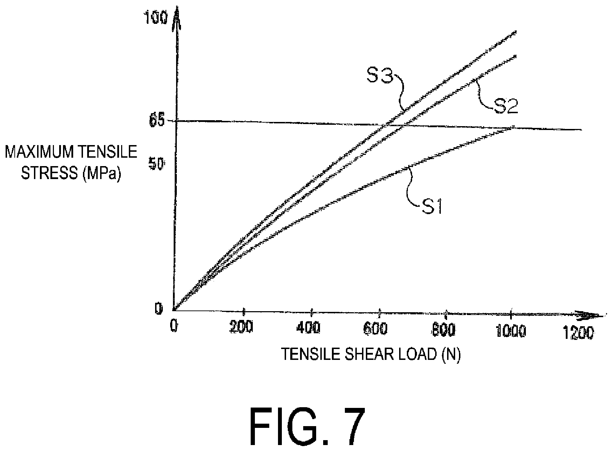

In addition, as shown in FIG. 7, in the course of the simulation, data of the maximum tensile stress generated in each of the analysis models 1M is acquired. FIG. 7 shows analysis data S1 of the analysis model 1Ma, analysis data S2 of the analysis model 1Mb, and analysis data S3 of the analysis model 1Mc. Here, when the allowable tensile stress of the other material 3, which has been determined as the one where the maximum tensile stress is generated, is set to 65 MPa, the tensile shear load being applied to the analysis model 1M is calculated for the analysis data S1, S2, and S3 by the computation device 7 at the time when the tensile shear load exceeds the allowable tensile stress of 65 MPa.

According to the analysis data S1, S2, S3 shown in FIG. 7, in the analysis models 1Ma, 1Mb, and 1Mc, the maximum tensile stress exceeding the allowable tensile stress of 65 MPa is generated in the other member 3 when the tensile shear load exceeds 1000 N, 680 N, and 640 N, respectively. Thus, it is determined that the analysis model 1Ma is least likely to experience adhesive failure (failure of the other member 3) and has the best bonding performance, followed by the analysis model 1Mb and the analysis model 1Mc in this order. As a result, the most suitable adhesive 4 to be selected for joining the one material 2 and the other material 3 is the adhesive 4a, followed by the adhesive 4b and the adhesive 4c in this order.

In addition to the selection criteria for the adhesive 4 described above, the suitable adhesive 4 can be selected among adhesives 4a, 4b, and 4c based on the maximum amount of deformation in each of the analysis models 1M generated during the simulation. In this case, the allowable amount of deformation (mainly the amount of tensile deformation) is set for the analysis model 1M, and the maximum amount of deformation (mainly the amount of tensile deformation) in the analysis model 1M, generated during the simulation when a tensile shear load is applied until it reaches the tensile shear load that the analysis model 1M can withstand, is calculated by the computation device 7. When the maximum amount of deformation generated in the analysis model 1M exceeds the allowable amount of deformation, the adhesive 4 used in the analysis model 1M is excluded from the candidates for selection.

When selecting the adhesive 4 to be used in the adhesion complex 1 for use in a limited peripheral space, the selection criterion based on the maximum amount of deformation can be adopted. The selection criterion based on the maximum amount of deformation may be applied after the application of the selection criterion based on the allowable tensile stress described above, which takes the highest priority.

The physical property data to be used to construct the analysis model 1M is generally data obtained at ambient temperature (approximately 15°C to 30°C); however, when data of temperature dependency is available, physical property data obtained at a temperature compatible with the use condition of the adhesion complex 1 may be used. When selection of the adhesive 4 takes into consideration the data of temperature dependency, it becomes easy to select a suitable adhesive 4 that accommodates the use condition of the adhesion complex 1. The selection criterion based on the data of temperature dependency may be applied after the application of the selection criterion based on the allowable tensile stress described above, which takes the highest priority, and the selection criterion based on the maximum amount of deformation, which takes the second highest priority.

As described above, according to the adhesive selection method, data of tensile stress generated in an analysis model 1M and the location of the tensile stress generated is used in the course of a simulation in which a tensile shear load is applied to the analysis model 1M, the analysis model 1M being constructed based on the test data acquired by performing a tensile shear test on the adhesion complex 1 until adhesive failure takes place. Thus, while the adhesive selection method adopts a simple simulation in which a tensile shear load is applied to the analysis model 1M, it also enables selection of the adhesive 4 while taking into consideration the process, experienced by the adhesion complex 1, which ends with adhesive failure. Then, by performing the simulation described above for a plurality of analysis models 1M (1Ma, 1Mb, and 1Mc) with varied adhesive 4 (4a, 4b, 4c), it is possible to suitably select, among the adhesives 4a, 4b, and 4c, an adhesive 4 that can ensure bonding performance that satisfies requirements.

When a three-dimensional analysis model 1M is used, it becomes easier to predict the actual bonding performance of the adhesion complex 1 with higher accuracy. Meanwhile, when a two-dimensional analysis model 1M is used, the time required for model construction can be significantly reduced. When the specification of the adhesion complex 1 has a uniform width that is sufficiently long, the suitable adhesive 4 can be selected even by using a two-dimensional analysis model 1M.

In the production method for the adhesion complex 1 according to an embodiment of the present invention, the one member 2A made of the one material 2 and the other member 3A made of the other material 3 are compression-bonded with the adhesive 4 selected according to the selection method described above interposed between the one member 2A and the other member 3A. Then, the adhesive 4 is hardened and cured until sufficient adhesive strength is expressed, integrating the one member 2A and the other member 3A. In this way, the adhesion complex 1 illustrated in FIG. 1 is obtained.

In the adhesion complex and the production method for an adhesion complex according to an embodiment of the present invention, a suitable adhesive 4 is used, making it possible to obtain an adhesion complex 1 suitable for the use condition. Accordingly, it becomes advantageous to improve durability of the adhesion complex 1 and trust in the durability.

### Examples

Adhesives 4a, 4b, and 4c that are test samples of the adhesive bonded article described in the embodiment described above were produced using compositions in which components of the main agent and the curing agent were blended in the blended amount (parts by mass) shown in Table 1, and test data was acquired. The physical property data of each of the adhesives 4a, 4b, and 4c is as described above. The results of simulation using the analysis model constructed based on the test data were shown in FIG. 7.

**[Table 1]**

| | | Adhesive | | |
|---|---|---|---|---|
| | | 4a | 4b | 4c |
| Main agent | Urethane prepolymer 1 | 42.0 | | |
| | Urethane prepolymer 2 | | 70.4 | |
| | Urethane prepolymer 3 | | | 53.0 |
| | Isocyanurate | | 1.5 | |
| | Carbon black | 21.8 | | |
| | Calcium carbonate 1 | 20.0 | 14.1 | 37.0 |
| | Silica | | 3.5 | |
| | Dehydrating agent | | | 10.0 |
| | Plasticizer | 16.0 | 10.6 | |
| | Catalyst 1 | 0.2 | | |
| | Total | 100.0 | 100.0 | 100.0 |
| Curing agent | Polyol 1 | 5.0 | 7.2 | |
| | Polyol 2 | | 46.3 | |
| | Polyol 3 | | 2.9 | 8.0 |
| | Polyol 4 | | | 9.9 |
| | Polyol 5 | | | 19.9 |
| | Polyol 6 | 47.9 | | |
| | Polyol 7 | | | 19.9 |
| | Calcium carbonate 2 | 46.8 | 43.5 | |
| | Talc | | | 41.6 |
| | Catalyst 2 | 0.3 | | |
| | Catalyst 3 | | 0.01 | 0.61 |
| | Total | 100.0 | 100.0 | 100.0 |
| Main agent/curing agent (mass ratio) | | 10/1 | 10/2.5 | 10/6.7 |

Details of each of the components in Table 1 are as follows.
- Urethane prepolymer 1: produced by mixing polyoxypropylene diol (SANNIX PP2000, available from Sanyo Chemical Industries, Ltd.; weight average molecular weight: 2000), polyoxypropylene triol (SANNIX GP3000, available from Sanyo Chemical Industries, Ltd.; weight average molecular weight: 3000), and MDI (methylene diphenyl diisocyanate) (Sumidur 44S, available from Sumika Bayer Urethane Co., Ltd.) in a manner that NCO/OH (molar ratio) was 2.0 and reacting the mixture at 80°C for 5 hours.
- Urethane prepolymer 2: produced by mixing polytetramethylene glycol (PTMG 650, available from Mitsubishi Chemical Corporation; weight average molecular weight: 650), polyoxypropylene diol (SANNIX PP2000, available from Sanyo Chemical Industries, Ltd.; weight average molecular weight: 2000), and MDI (methylene diphenyl diisocyanate) (Sumidur 44S, available from Sumika Bayer Urethane Co., Ltd.) in a manner that NCO/OH (molar ratio) was 2.0 and reacting the mixture at 80°C for 5 hours.
Urethane prepolymer 3: produced by mixing polyoxypropylene ethylene diol (EXCENOL 510, available from AGC Chemicals; weight average molecular weight: 4000), polymeric MDI (Millionate MR-200, available from Tosoh Corporation), and carbodiimide-modified MDI (Millionate MTL, available from Tosoh Corporation) in a manner that NCO/OH (molar ratio) was 35.0 and reacting the mixture at 80°C for 5 hours.
- Isocyanurate: isocyanurate of pentamethylene diisocyanate
- Carbon black: #200 MP, available from NSCC Carbon Co., Ltd.
- Calcium carbonate 1: SUPER S (heavy calcium carbonate), available from Maruo Calcium Co., Ltd.
- Silica: AEROSIL R972 (surface-treated fumed silica), available from Nippon Aerosil Co., Ltd.
- Dehydrating agent: Zeosil A-4 (zeolite), available from Solvay Japan
- Plasticizer: DINP (diisononyl phthalate), available from J-PLUS Co., Ltd.
- Catalyst 1: UCAT-660M (DMDEE, dimorpholinodiethylether), available from San-Apro Ltd.
- Polyol 1: EXCENOL 450ED (polyoxypropylene tetraol with EO terminal; weight average molecular weight: 500), available from Asahi Glass Co., Ltd.
- Polyol 2: PTMG-2000 [polytetramethylene glycol(diol), weight average molecular weight: 2000], available from Mitsubishi Chemical Corporation
- Polyol 3: 14BG (1,4-butanediol, molecular weight: 90) available from Mitsubishi Chemical Corporation
- Polyol 4: UNIOL D-400 [polyoxypropylene glycol(diol), weight average molecular weight: 400], available from NOF Corporation
- Polyol 5: EXCENOL 3020 [polypropylene glycol(diol), weight average molecular weight: 3000], available from AGC Chemicals
- Polyol 6: PREMINOL7001K (polyoxypropylene triol with EO terminal, weight average molecular weight: 6500), available from Asahi Glass Co., Ltd.
- Polyol 7: EXCENOL 823 [polypropylene ethylene glycol(triol), weight average molecular weight: 5000), available from AGC Chemicals
- Calcium carbonate 2: KALFAIN 200 (calcium carbonate subjected to surface treatment with fatty acid), available from Maruo Calcium Co., Ltd.
- Talc: Soap Stone A (average particle size: 3.5 to 4.0 µm; aspect ratio: 9.5), available from Nippon Mistron Co., Ltd.
- Catalyst 2: U-810 (dioctyltin dilaurate), available from Nitto Kasei Co., Ltd.
- Catalyst 3: DABCO 33-LV (33% solution of DABCO in propylene glycol), available from Sigma-Aldrich

The test samples were produced as follows.

### • Surface treatment step

Each member (resin base material) was surface-treated prior to the application of adhesive to the surface of each member. The surface treatment (frame treatment) was performed using a frame treatment apparatus available from Arcogas (gas flow rate: 3.7 L/min; air flow rate: 100 L/min) at a treatment speed of 800 mm/sec, a distance to member of 20 mm, and a number of times of one.

Here, "treatment speed" refers to the speed of the flame treatment, and specifically, the speed (mm/sec) of moving a frame treatment apparatus with respect to a member. "Distance to member" refers to the distance (mm) between a frame treatment apparatus and a member. "Number of times" refers to the number of times of sweeping of the flame. For example, when the frame is swept one time from one end to the other end of a member, the number of times is one.

### • Adhesive layer forming step

The urethane-based adhesives (adhesives 4a, 4b, 4c) described above were applied to the treated surface of the one member to form an adhesive layer.

### • Bonding • curing step

Furthermore, the treated surface of the other member was adhered to the surface at which the adhesive layer was formed to form a multilayer structure. Next, the multilayer structures were left to stand for three days at 23°C and 50% RH to cure the adhesive layer, thus preparing test samples.

As can be seen from the analysis results in FIG. 7, a lower tensile elastic modulus of an adhesive indicates a smaller maximum stress generated in an analytical model, and thus indicates a higher tensile shear load that the analytical model 1M can withstand. This is thought to be because: when the tensile elastic modulus of an adhesive is high, the function of alleviating the stress generated is suppressed; as such, stress is concentrated in the material with higher rigidity, leading to failure. Note that the analysis results in FIG. 7 substantially match the test results of each of the test pieces.

### Reference Signs List

1 Adhesion complex
1M (1Ma, 1Mb, 1Mc) Analysis model
2 One material
2A One member
3 The other material
3A The other member
4 (4a, 4b, 4c) Adhesive
5 Shear tester
6a, 6b Holder
7 Computation device

## Claims

1. An adhesive selection method, comprising:
acquiring test data by subjecting an adhesion complex, obtained by integrally bonding one material and an other material that are heterogeneous using an adhesive, to a shear test, in which a tensile shear load is applied to the adhesion complex in a direction that causes shear deformation in the adhesive, until adhesive failure takes place;
constructing, based on the test data, a plurality of two-dimensional or three-dimensional FEM analysis models of the adhesion complex with varied adhesives;
performing, on each of the plurality of analysis models, a simulation in which a tensile shear load is applied in a direction that causes shear deformation in the adhesive used in the analysis model; and
selecting, from the adhesives, an adhesive for bonding the one material and the other material based on the tensile stresses generated in the analysis models in the course of the simulation and the locations where the tensile stresses are generated.

2. The adhesive selection method according to claim 1, wherein
the adhesive for bonding the one material and the other material is selected, among the adhesives, based on a comparison between a maximum tensile stress in each of the one material, the other material, and the adhesive of the analysis model generated in the course of the simulation and an allowable tensile stress of each of the one material, the other material and the adhesive.

3. The adhesive selection method according to claim 1, wherein
the adhesive for bonding the one material and the other material is selected, among the adhesives, based on a comparison between a maximum tensile stress in the analysis model generated in the course of the simulation and an allowable tensile stress of a member having a location where the maximum tensile stress is generated.

4. The adhesive selection method according to any one of claims 1 to 3, wherein
the adhesive for bonding the one material and the other material is selected, among the adhesives, based on a maximum amount of deformation in the analysis model generated in the course of the simulation.

5. The adhesive selection method according to claim 4, wherein
the adhesive for bonding the one material and the other material is selected, among the adhesives, based on a comparison between an allowable amount of deformation of each of the analysis models and the maximum amount of deformation.

6. The adhesive selection method according to any one of claims 1 to 5, wherein:
the one material has a tensile elastic modulus of 500 MPa or greater and 2000 MPa or less and a tensile strength of 10 MPa or greater and 30 MPa or less;
the other material has a tensile elastic modulus of 2000 MPa or greater and 10000 MPa or less and a tensile strength of 30 MPa or greater and 150 MPa or less;
the tensile elastic modulus of the other material is two-times or greater the tensile elastic modulus of the one material; and
the plurality of adhesives has a tensile elastic modulus of 1 MPa or greater and 25 MPa or less and a tensile strength of 3 MPa or greater and 25 MPa or less.

7. The adhesive selection method according to any one of claims 1 to 6, wherein:
a main component of the one material and the other material is polyurethane; and
the adhesive is a two-part polyurethane-based adhesive comprising a main agent containing a urethane prepolymer and a curing agent containing a polyol.

8. An adhesion complex, comprising:
an adhesive selected in accordance with the adhesive selection method according to any one of claims 1 to 7;
one member made of the one material; and
the other member made of the other material;
the adhesive being interposed between the one member and the other member and integrating the one member and the other member.

9. A production method for an adhesion complex, comprising:
interposing an adhesive selected in accordance with the adhesive selection method according to any one of claims 1 to 7 between one member made of the one material and the other member made of the other material to integrate the one member and the other member.
